# EUROPÄISCHE PATENTANMELDUNG

(11) **EP 1 302 157 A2**
(43) Veröffentlichungstag der Anmeldung: **16.04.2003**
(21) Anmeldenummer: 02022414.3
(22) Anmeldetag: 04.10.2002
(51) Int. Cl.: A61B 5/03

(54) **Vorrichtung zur Bestimmung des intrazerebralen Druckgradienten**

(30) Priorität: 13.10.2001 DE 20116879 U
(71) Anmelder: REHAU AG + Co, 95111 Rehau (DE)
(72) Erfinder: Gropp, Friedrich, c/o Rehau AG + Co, 95111 Rehau (DE); Kunze, Gerd, c/o Rehau AG + Co, 95111 Rehau (DE); Hoell, Thomas, 06112 Halle (DE)

(57) **Zusammenfassung**

Vorrichtung zur Messung des Hirndruckes mittels Katheter, wobei der Katheter mindestens aus einem Katheterschlauch aus polymeren Material besteht und über mindestens zwei Drucksensoren verfügt, die in Reihe angeordnet sind, wobei der eine Drucksensor den Hirndruck in der geschädigten Region, der zweite oder weitere Drucksensoren den Hirndruck in den angrenzenden Bereichen erfassen und dass die Plazierung des Katheters und die Messung des Hirndruckes an beliebigen Stellen im Hirn erfolgt.

## Beschreibung

Der Stand der Technik bei der Messung des intrazerebralen Druckes ist in den Hirndruckdruckmesssonden "Neurovent" der REHAU AG + Co samt Zubehör aktuell dargestellt. Hier finden alle üblichen Mess- und Applikationstechniken Anwendung, die benötigt werden, um einen mit einem Sensor versehenen Katheter nach den gebräuchlichen Regeln an den vorgesehenen Orten zu platzieren, um die gewünschten Messwerte für den Hirndruck zu erhalten, sowie grafisch darstellen zu können.
Dazu wird - nach der bisher geübten medizinischen Praxis - der Katheter meistens im Bereich des vorderen Schädels, rechts oder links frontal, eingeführt und der Hirndruck an folgenden Messorten bestimmt:
1. Auf der harten Hirnhaut
2. Im Hirngewebe
3. In den Hirnwasserkammern (Ventrikeln)

Hier geht man von der im vielen Fällen unzutreffenden, aber medizinisch akzeptierten Annahme aus, dass der intrazerebrale Druck im Hirn an allen Punkten etwa in gleicher Größe anliegt. Für gewöhnlich wird daher der Hirndruck nur durch einen Einzelmesspunkt erfasst.

Es ist jedoch auch bekannt, dass das menschliche Hirn unter bestimmten Bedingungen an verschiedenen Messorten Druckdifferenzen, sog. Druckgradienten, aufweisen kann. Daher wird in Ausnahmefällen mit zwei Sensoren gearbeitet, von denen jeder in einem gesonderten Katheter platziert ist. Dies wird überwiegend im Rahmen von Studien durchgeführt und ist in der klinischen Praxis nicht üblich.

Die wünschenswerte Lage des Messsensors in unmittelbarer Nähe zur pathologischen Veränderung ist also in seltenen Fällen - und nur zufällig - zu erreichen. Da bisher nur der allgemeine Hirndruck erfasst werden konnte, war die Platzierung an den quasi genormten Messorten üblich.

Besonders nachteilig ist, dass ohne Patientenrisiko eine Lagekorrektur des Sensors nachträglich nicht möglich ist. Vielmehr muss der Katheter komplett entfernt und ein neuer gelegt werden, um die Sterilitätsbedingungen zu sichern.
Nachteilig ist darüber hinaus, dass mehrere Sensoren nicht über einen Zugangskanal und in variablen Abständen positionierbar sind. Wenn mehrere Sensoren zum Einsatz kommen müssen, so geschieht dies in der Regel zwar über eine Kopfschraube, jedoch über mehrere Katheter und damit über mehrere Zugangskanäle in der Hirnhaut.

Aus diesen Nachteilen des Standes der Technik ergibt sich die Aufgabe, eine Vorrichtung bereit zu stellen, welche diese Negativpunkte überwindet und insbesondere die Möglichkeit bereit stellt, dass mindestens zwei Hirndruckmesssensoren über einen Zugang gleichzeitig an mindestens zwei verschiedenen Punkten im Hirn platziert werden können.

Das geschieht erfindungsgemäß durch ein Kathetersystem, in dem mindestens zwei Sensoren in Reihe angeordnet sind, wobei mindestens ein Sensor den Hirndruck in der geschädigten Hirnregion und der zweite oder weitere im angrenzenden Bereich des Hirns den Hirndruck erfassen.
Die Positionierung geschieht vorzugsweise über Neuronavigation oder Ultraschall, wobei entweder über eine separate Hülse in Verbindung mit einem eingebrachten Mandrin der Zugangskanal erzeugt wird und anschließend das Kathetersystem eingebracht wird, oder das Kathetersystem enthält einen Mandrin und verfügt über eine atraumatische Katheterspitze, so dass es direkt eingeführt und positioniert werden kann.

Die Messung der absoluten Werte der Hirndrücke, sowie der zeitliche Verlauf der Differenzwerte, können nun die Grundlage für eine wesentlich wirksamere Therapiestrategie bilden, als dies bisher möglich war.

Alle Materialien sind CT-, MRT- und röntgentauglich, d. h. aus unmagnetischen Materialien, wie z. B. Titan, Keramik oder hochpolymeren Werkstoffen gefertigt.

Nachfolgend soll die Erfindung und einige Variationsmöglichkeiten davon, die nicht auf die beschriebenen Ausführungsbeispiele beschränkt sind, näher erläutert werden.

Erfindungsgemäß lässt sich beispielsweise eine erste Hirndruckmesssonde in einem einlumigen Katheter mit zwei piezoelektrischen Sensoren zur Hirndruckmessung herstellen. Dabei ist der erste Sensor an der Spitze des Katheterschlauches, der zweite im Abstand dahinter im selben Lumen angeordnet. Der Katheterschlauch selbst besteht aus Polyurethan, das auf übliche Weise, beispielsweise mit Zusatz von Wolframpulver, röntgen-, CT- und MRT-kontrastfähig ausgerüstet ist.
Weiterhin ist der Katheterschlauch signalfarben orange eingefärbt, damit er - bei dem geringen Durchmesser von 2 mm - bei der Operation leicht erkennbar und identifizierbar ist.

Nach dem Öffnen des Schädels nahe dem geschädigten Bereich wird, wie üblich, der Bolt-Kit gesetzt und über ein System mit Hülse und Mandrin der mit den Sensoren bestückte Teil des erfindungsgemäßen Katheters im Kopf platziert. Das geschieht über ein Neuronavigationssystem oder mittels Ultraschall und zwar so, dass im geschädigten Hirnbereich ein Sensor, ein zweiter Sensor in der gesunden Hirnmasse oder im Ventrikel platziert werden.
Die Sensoren sind über ein entsprechendes Adaptersystem an den Messmonitor gekoppelt, die so zugänglichen Druckgradienten der einzelnen Messstellen und deren zeitliche Veränderung lassen sich nun abrufen und dokumentieren.
Um beim Implantieren des Katheters die einzelnen Abstände der Sensoren zu erkennen, ist der Katheter zirkulär mit einer Skalierung und Beschriftung versehen.

Eine zweite Möglichkeit, die Erfindung auszugestalten, geht von einem zweilumigen System mit zwei Drucksensoren zur Hirndruckmessung aus, wobei ein großlumiger Katheterschlauch an seinem distalen Ende über einen Drucksensor und eine axiale Öffnung verfügt, in die ein eingebrachter kleinlumiger Katheterschlauch, versehen an seinem distalen Ende mit einem Drucksensor, hineinragt. Beide distale Sensoren bilden in ihrer Ausgangsposition eine atraumatische Katheterspitze. Auch diese Anordnung wird beispielsweise über eine Hülse mit innenliegendem Mandrin positioniert. Beide Katheterschläuche bestehen jeweils aus einem Verbundschlauch, wobei dessen innere Schicht aus Polyethylen, dessen äußere Schicht aus Silikonkautschuk besteht. Die beiden Katheterschläuche sind ebenfalls CT-, MRT- und röntgenkontrastfähig ausgerüstet und unterschiedlich signalfarben eingefärbt.
Die Lumen beider Katheterschläuche sind über ein proximal montiertes Ventil gekoppelt, welches mit dem großlumigen Katheterschlauch fest montiert ist und in dessen Lumen der kleinlumige Katheterschlauch durch eine Quetschdichtung dicht und axial fixiert ist.

Im Falle einer erforderlichen Korrektur des Abstandes der Sensoren wird nun die Dichtung geöffnet, der erforderliche Abstand hergestellt - kontrolliert durch die Markierungen der Katheterschläuche - und die Dichtung wieder geschlossen.
Der bewegte Abschnitt des kleinlumigen Katheterschlauches ist für diese Handhabung mit einer flexiblen Schlauchschutzhülle umgeben, welche die sterile Oberfläche des bewegten Schlauchabschnittes während der notwendigen Positionsveränderung sichert.
Alternativ ist es auch möglich, den großlumigen Katheterschlauch über einen atraumatischen, starren Mandrin vorab zu implantieren und nach dem Entfernen dieses Mandrins den kleinlumigen Katheterschlauch zu installieren.

Eine weitere Möglichkeit der erfindungsgemäßen Ausführung sei hier noch erwähnt. Hier sind beide Sensoren in dem einem Lumen des Katheters in Reihe platziert, in einem zweiten Lumen des Katheters befindet sich ein starrer Mandrin, der es ermöglicht, den Katheter sicher zu stabilisieren und ohne Abweichung zu führen. Dabei ist die Mandrinspitze so in der Katheterspitze zentral angeordnet, dass es zu keiner axialen Abweichung durch Schub während der Implantation kommen kann.

Die Vorteile des erfindungsgemäßen Kathetersystems sind vielfältig. Zunächst ist es möglich, dass die Messung am Ort der Hirnverletzung und parallel im Bereich des umgebenden Gewebes erfolgen kann. Damit ist eine konkrete Aussage zu den tatsächlichen Druckverhältnissen und besonders zum Druckgradienten möglich. Weiterhin ist nur ein Zugangskanal nötig, um eine Messung an mehreren Punkten mit mehreren Sensoren durchführen zu können. Besonders vorteilhaft ist, dass die Sensoren präzise positionierbar sind, sowohl im klassischen, manuellen Handling, wie auch mit Hilfe der Neuronavigation oder des Ultraschalls. Dies ist möglich entweder durch den Einsatz eines starren Mandrins im Katheter (sh. Beispiel 2) oder durch die Platzierung des Katheters nach dem Einbringen des Zugangskanals über die Kombination Hülse/Mandrin.
Nicht zuletzt ist die Kombination mit gebräuchlichen Hilfsmitteln gesichert, z.B. mit Tunnelungshülsen oder Kopfschrauben oder lumengeführten starren Führungsdrähten.

Neben den Vorteilen bei der Platzierung sind die messtechnischen Vorteile bei der Anwendung des erfindungsgemäßen Kathetersystems erheblich.

Zunächst ist es vorteilhaft, dass die Diagnostik der Hirndruckverläufe im geschädigten Hirnbereich im direkten Vergleich mit dem Hirndruck im umgebenden, gesunden Gewebe erfolgt und damit lokal bewertbar ist. Damit wird erstmals in der Hirndruckdiagnose der Vergleich von lokalem und allgemeinem Hirndruck möglich, wobei vorzugsweise der sog. Goldstandard des Hirndruckes, gemessen im Ventrikel, als allgemeiner Hirndruck im Sinne der bestmöglichen Bezugsgröße, angestrebt wird. Die Einzelerfassung der Messdaten je Sensor, sowie deren Verarbeitung z. B. in Form von Druckdifferenzwerten, führt zu Ergebnisdarstellungen, die zielgerichtet den Outcome der Patienten verbessern.
Weiterhin ist die Erfassung und Auswertung der Messergebnisse einfach und den bisherigen Verfahrensweisen angepasst. Es ist keine zusätzliche Hardware nötig und nur geringfügige Aufwendungen an Software für die vorhandenen Monitorsysteme.
Für die Diagnose ist besonders vorteilhaft, dass, wie im Beispiel 2 beschrieben, die Lage des zweiten Sensors in der Längsachse des Katheters veränderbar ist.

Die aufgeführten Vorteile ergeben also letztlich optimale Behandlungszeiträume durch frühzeitige Einleitung in Therapiemaßnahmen sowie sinkende Belastungen für Arzt und Patienten und damit auch eine entsprechende Kostenoptimierung.

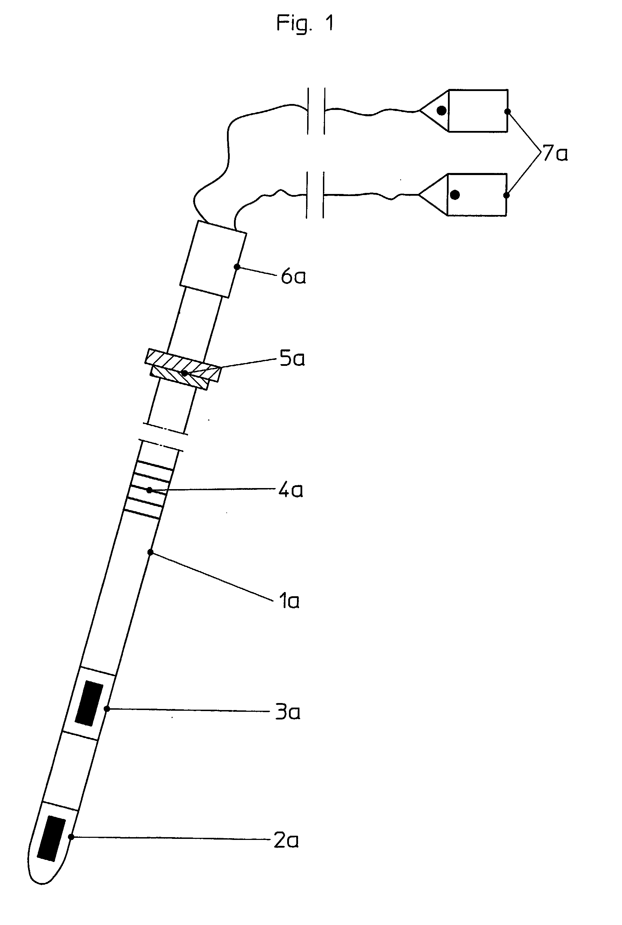

Im Folgenden wird anhand von zwei Figuren die Erfindung näher erläutert.
Dabei zeigt
- Figur 1 die erfindungsgemäße Vorrichtung in der Ausführung eines Einlumen-Katheters

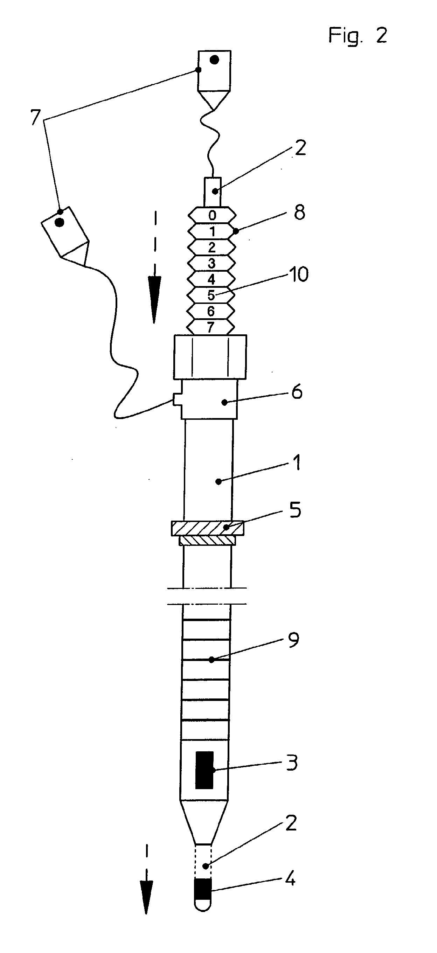

- Figur 2 die erfindungsgemäße Vorrichtung in der Ausführung eines Zweilumen-Katheters

### Figur 1

zeigt eine einlumige Ausführung der Erfindung.
In den Katheterschlauch 1a sind zwei Sensoren 2a, 3a zur Hirndruckmessung integriert, wobei der erste Sensor 2a am distalen Ende und der zweite Sensor 3a proximal dahinter angeordnet sind. Die Sensoren befinden sich in Gehäusen aus nicht magnetisierbarem Material, hier Keramik.

Die Skalierung 4a ist MRT-kontrastgebend, hier titanfarben, ausgeführt und ringförmig auf eine Länge von 20 cm angebracht. Der Abstand der Markierungen beträgt je 1 cm. Die Sensoren 2a, 3a sind über eine Weiche 6a mit den Steckern 7a verkabelt. Der Schlauchreiter 5a dient als Abstandshalter oder zur Fixierung der Eindringtiefe des Katheters.

### Figur 2

zeigt eine zweilumige Ausführung der Erfindung, wobei - für viele Fälle besonders vorteilhaft - eine Anordnung gewählt wurde, die es ermöglicht, beide Sensoren axial gegen einander zu bewegen: Der großlumige Katheterschlauch 1 verfügt am distalen Ende über eine axiale Öffnung, proximal dahinter folgt ein Sensor 3, ein Schlauchreiter 5 und die ringförmige Skalierung 9 in Abständen von jeweils 1 cm. Das Ventil 6 ist als Endstück gestaltet, in welchem durch einen seitlichen Lumenausgang des Ventils 6 die elektrische Verkabelung des Sensors zum Monitor abzweigt und im zentralen Lumen des Ventils 6 der kleinlumige Katheterschlauch 2 oder unter Umständen ein starrer Mandrin positioniert sind.

Der kleinlumige Katheterschlauch 2 verfügt am distalen Ende über eine konisch ausgebildete Spitze mit Sensor 4.
Die beiden axial angeordneten Katheterschläuche bilden aus dem Sensorgehäuse des kleinlumigen Katheterschlauches 2 und der konischen Spitze des großlumigen Katheterschlauches 1 eine atraumatische Spitze, die eine präzise Führung und Platzierung des Systems im Hirn ermöglicht. Ist die Positionierung erfolgt, so kann das Ventil 6 geöffnet und die Sensoren 3, 4 gegeneinander axial verschoben werden. Diese Bewegung ist über die Skalierungen 9, 10 kontrolliert verfolgbar. Mit dem Verschluss des Ventils 6 und der damit erfolgten Fixierung des kleinlumigen Katheterschlauches 2 können die Stecker 7 an die Messgerätetechnik gekoppelt werden, wobei die Übertragung der Messsignale an den Monitor über Kabel, oder - wenn der Monitor entsprechend ausgestattet ist - telemetrisch, beispielsweise über Funk, erfolgt.
Korrekturen der Sensorposition sind auch während der Messung möglich.
Zu Sicherung der sterilen Oberfläche des bewegten Schlauchabschnittes des Katheterschlauches 2 ist eine Schutzhülle 8 mit einem Ende am Ventil 6 und dem anderen Ende am Katheterschlauch 2 fest montiert, die vorzugsweise als Faltenbalg oder Folienschlauch aus transparentem, flexiblen Polymermaterial gestaltet ist.

## Patentansprüche

1. Vorrichtung zur Messung des Hirndruckes mittels Katheter,
**dadurch gekennzeichnet,**
**dass** der Katheter mindestens aus einem Katheterschlauch aus polymerem Material besteht, und über mindestens zwei Drucksensoren verfügt, die in Reihe angeordnet sind, wobei der eine Drucksensor den Hirndruck in der geschädigten Region, der zweite, oder weitere Drucksensoren den Hirndruck in den angrenzenden Bereichen erfassen und dass die Platzierung des Katheters und die Messung des Hirndruckes an beliebigen Stellen im Hirn erfolgt.

2. Vorrichtung gemäß Anspruch 1, **dadurch gekennzeichnet, dass** der Katheter aus einem großlumigen Katheterschlauch, der an seinem distalen Ende über einen Drucksensor und eine axiale Öffnung verfügt, und aus einem darin eingebrachten, kleinlumigen Katheterschlauch besteht, der an seinem distalen Ende mit einem Drucksensor versehen ist und der in die axiale Öffnung des großlumigen Katheterschlauches so hineinragt, dass beide Sensoren in ihrer Ausgangsposition eine atraumatische Katheterspitze bilden.

3. Vorrichtung gemäß Anspruch 1, **dadurch gekennzeichnet, dass** der Katheter über zwei Lumen verfügt, wobei in dem einen Lumen in Reihe Drucksensoren angeordnet sind, und in einem zweiten Lumen ein starrer Mandrin so platziert ist, dass mit dessen Hilfe das Kathetersystem präzise positionierbar ist.

4. Vorrichtung nach Anspruch 1, **dadurch gekennzeichnet, dass** für alle Elemente, die während der bildgebenden Diagnostik am Patienten verbleiben, unmagnetische Materialien zum Einsatz kommen.

5. Vorrichtung nach den Ansprüchen 1 und 4, **dadurch gekennzeichnet, dass** die unmagnetischen Materialien ausgewählt sind aus der Gruppe Keramik und/oder Polymere und/oder Titan.

6. Vorrichtung gemäß Anspruch 2, **dadurch gekennzeichnet, dass** der bewegliche, kleinlumige Katheterschlauch durch eine Schutzhülle aus flexiblem, transparentem Material gegen äußere Einflüsse geschützt ist.

7. Vorrichtung nach einem der vorigen Ansprüche, **dadurch gekennzeichnet, dass** die Katheterschläuche im nicht implantierten Bereich im erforderlichen Maße zirkulär skaliert sind und diese Skalierung mit Abstandsziffern versehen ist.

8. Vorrichtung nach einem der vorigen Ansprüche, **dadurch gekennzeichnet, dass** die Druck-Sensoren phasengleich arbeiten und das Messsignal ungedämpft an den Empfänger übertragen.

9. Katheter nach einem der vorigen Ansprüche, **dadurch gekennzeichnet, dass** die präzise Platzierung im Hirn mittels Mandrin, insbesondere mittels Hülsen-Mandrin-System erfolgt und die Platzierung über bildgebende Verfahren überwacht wird.

10. Katheterschlauch nach Anspruch 1, **dadurch gekennzeichnet, dass** das polymere Material signalfarben eingefärbt, sowie CT-, MRT- und röntgentauglich ist.

11. Katheterschlauch nach den Ansprüchen 1 und 10, **dadurch gekennzeichnet, dass** der Werkstoff ausgewählt ist aus der Gruppe Silikonkautschuk und/oder Polyurethan und/oder Polyamid und/oder Polyolefin.
